# EUROPÄISCHE PATENTSCHRIFT

(11) **EP 0 522 341 B1**
(45) Veröffentlichungstag und Bekanntmachung des Hinweises auf die Patenterteilung: **11.12.1996**
(21) Anmeldenummer: 92110471.7
(22) Anmeldetag: 22.06.1992
(51) Int. Cl.: C08L 83/07, A61K 6/10

(54) **Transparentes Material für dentale Anwendungen**
Transparent material for dental purposes
Produit transparent pour usage dentaire

(30) Priorität: 05.07.1991 DE 4122310
(43) Veröffentlichungstag der Anmeldung: 13.01.1993
(73) Patentinhaber: BAYER AG, 51368 Leverkusen (DE)
(72) Erfinder: Voigt, Reiner, Dipl.-Ing., W-5090 Leverkusen 1 (DE); Schulz, Hans-Hermann, W-5000 Köln 80 (DE); Wrobel, Dieter, Dr., W-5090 Leverkusen 1 (DE)

(56) Entgegenhaltungen:
- EP-A- 0 169 365
- DE-A- 2 535 334
- DE-A- 2 646 726
- FR-A- 2 277 848

## Beschreibung

Die vorliegende Erfindung betrifft ein transparentes Material auf Basis additionsvernetzender Polysiloxane, welches z.B. als Okklusalstempel zur Formung von Seitenzahncompositen oder als Fixierungsschiene für Brackets in der Kieferorthopädie sowie bevorzugt zur Bißregistherung im Dentalbereich Anwendung findet. Es ist in vielen Fällen wünschenswert, in zahnärztlichen und zahntechnischen Anwendungen transparente Materialien einzusetzen, die eine Kontrolle von Einstellungen oder Positionierungen aufgrund ihrer Durchsichtigkeit erlauben und Fixation durch dieses Material, z. B. durch Lichtpolymerisation, ermöglichen.

Die als bevorzugt genannten Bißregistriermaterialien in der Zahnheilkunde werden eingesetzt, um die richtige Lagebeziehung von Unterkiefer zu Oberkiefer im Mund zu fixieren und diese Position im zahntechnischen Laboratorium auf die Modellle zu übertragen. Verwendet werden hierfür Wachse und bevorzugt elastomere Materialien wie Polyether und Silikone. Bißregistriermaterialien müssen besonderen Anforderungen genügen. Sie sollten eine standfeste Konsistenz aufweisen und sich durch hohe Endhärte, geringe elastische Verformbarkeit und eine minimale Dimensionsveränderung auszeichnen. Das erfindungsgemäße Material auf Basis additionsvernetzender Silikonmassen zeichnet sich durch hohe Transparenz aus, die im Mund die Kontrolle der vom Patienten eingenommenen Zahnposition ermöglicht und ggf unter Sichtkontrolle Korrekturen der Bißlage gestattet. Darüberhinaus kann das Material auf sehr kurze Verarbeitungszeit eingestellt werden. Eine Verarbeitungszeit von nur ca. 35 - 45 Sekunden reduziert das Risiko der Lageverschiebung des Unterkiefers aus der einmal eingenommenen Position durch den Patienten. Gemessen vom Mischbeginn kann z. B. der Quetschbiß nach 2 Minuten 30 Sekunden dem Mund entnommen werden. Nach Ende der Abbindezeit, also nach Herausnahme aus dem Mund, erleidet dieses additionsvernetzende Silikonmaterial keine klinisch relevanten Dimensionsänderungen und gibt damit Bißregistrate, die dauerhaft lagerstabil sind. Von besonderer Bedeutung ist auch auf Grund der Transparenz die Kontrollmöglichkeit der Modellposition im Bißregistrat. Positionsverschiebungen der Modelle, die zu einem zeitaufwendigen Korrigieren der fertigen Arbeit im Mund führen, können durch diese Kontrollmöglichkeit, die bei allen übrigen undurchsichtigen Materialien nicht gegeben ist, schon vor Anfertigung des Zahnersatzes festgestellt werden.

Dental-Abformmassen auf Basis additionsvernetzender Polysiloxane sind an sich bekannt (vgl. z. B. R: G. Graig, Restaurative Dental Materials, The C. V. Moosbe-Comp., St. Louis, 1980, S. 195 ff).

Im allgemeinen bestehen diese Massen aus einer Silikonöl, Füllstoff und Vernetzer enthaltenden Basispaste und einer Katalysatorpaste aus Silikonöl, Füllstoff und Katalysator.

Bei Anwendung werden die Basis- und Katalysatorpaste gewichts- oder volumenmäßig vorzugsweise 1:1 gemischt und mittels Spritze und/oder Abformlöffel an die abzuformende Kieferpartie appliziert. Nach dem Vernetzen durch eine Polyadditionsreaktion wird die gummiartige Abformung aus dem Mund des Patienten entfernt und anschließend mit einer wässrigen Aufschlämmung von Modellgips ausgegossen.

Auch Bißregistriermaterialien auf Basis additionsvernetzender Silikone sind bekannt und im Handel erhältlich. Sie sind ähnlich aufgebaut wie die o. g. Dental-Abformmassen. Die höheren Endhärten und geringeren elastischen Verformungen werden durch den Einsatz noch größerer Anteile von Füllstoffen erzielt. Die kurzen Verarbeitungszeiten dieser Massen lassen sich durch einen höheren Platin-Katalysator-Anteil einstellen. Diese hochgefüllten Zwei-Komponenten-Materialien sind undurchsichtig und geben nach der Additionsreaktion einen harten und spröden Gummi.

Aus EP-A-0 169 365 sind Compounds aus hochdispersen aktiven Füllstoffen und Silikonpolymeren zur Herstellung von Abformungen sowie zur Vervielfältigung von Gipsmodellen bekannt geworden. Diese Produkte sind jedoch nicht transparent.

Aus US-Patent 4,571,188 ist eine Abdeckmatrix für lichtvernetzbare Füllstoffe bekannt geworden, die aus 6 % Polymethylhydro (65 - 70 %) dimethylsiloxan-Copolymer und 94 % Vinyldimethyl endgestopptem Polydimethylsiloxan einer Viskosität von 60.000 csk besteht, wobei als Katalysator eine Mischung von 1 % Chloroplatinsäurekomplex und 99 % Vinyldimethyl endgestopptem Polydimethylsiloxan einer Viskosität von 60.000 csk eingesetzt wurde. Es wird gelehrt, Basispaste und Katalysatorpaste mit einem Spatel zu mischen und das erhaltene Material auf die zu restaurierende Zahnoberfläche und den umgebenden Bereich aufzubringen. Nach dem Vernetzen der Mischung soll die erhaltene Oberflächenreproduktion wieder entfernt werden und die Kavität mit einem lichtvemetzenden Komposit versorgt werden. Im Anschluß daran soll die zuvor entfernte Oberflächenreproduktion replaziert werden und das lichtvernetzende Komposit bestrahlt werden. Wie jedoch die in der vorliegenden Patentanmeldung enthaltenen Vergleichsversuche zeigen, ist das dort offenbarte Material zur Erzeugung der angesprochenen Oberflächenreproduktion zum diesem Zweck völlig ungeeignet, da die mechanische Festigkeit des Materials hierzu nicht ausreicht.

Durch den erfindungsgemäßen Einsatz von Compounds von hochdispersen aktiven Füllstoffen in Silikonölen, welche in der DE-A-25 35 334 beschrieben sind, und durch den erfindungsgemäßen Einsatz einer kurzkettigen vinylgruppenreichen Polysiloxan-Verbindung sowie ggf. eines kurzkettigen vinylgruppenhaltigen QM-Harzes erhält man hochviskose, transparente Pasten, die zu harten Gummis mit guter Reißfestigkeit und hoher Transparenz in sehr kurzer Zeit vernetzen.

Diese QM-Harze sind dadurch gekennzeichnet, daß sie, wie in W. Noll, Chemie und Technologie der Silikone, Verlag Chemie, Weinheim, 2. Auflage, 1964, Seite 3 erklärt, als Q-Einheiten das tetrafunktionelle SiO₄/₂ und als M-Bausteine das monofunktionelle R₃SiO₁/₂, wobei R Vinyl-, Methyl-, Ethyl-, Phenyl sein kann, enthalten. Darüber hinaus können auch noch als T-Einheiten das trifunktionelle RSiO₃/₂ und als D-Einheiten das bifunktionelle R₂SiO₂/₂ mit der gleichen Bedeutung für R wie oben vorhanden sein. Der erfindungsgemäße Gehalt von 0 - 10 Gew.-% QM-Harz, vorzugsweise 0 - 5 Gew.-% QM-Harz im gesamten Silikonsystem bewirkt eine deutliche Verbesserung der Vernetzungsdichte und damit eine höhere Reißfestigkeit und Härte des elastomeren Produktes.

Gegenstand der Erfindung sind somit bei Umgebungstemperatur härtbare Massen auf Polysiloxanbasis, die nach dem Additionsverfahren vernetzen, enthaltend
a) Organopolysiloxane mit zwei oder mehr Vinylgruppen im Molekül,
b) Organohydrogenpolysiloxane mit zwei oder mehreren Si-H-Gruppen im Molekül,
c) einem Katalysator zur Beschleunigung der Additionsreaktion und gegenfalls
d) Farbstoffe,
dadurch gekennzeichnet, daß die Massen zusätzlich
e) Compounds von hochdispersen, aktiven Füllstoffen in Silikonöl,
f) kurzkettige Organopolysiloxane mit zwei oder mehreren Vinylgruppen im Molekül und
g) gegebenenfalls ein niedermolekulares, Vinyl- und Ethoxygruppen-haltiges, in a) homogen lösliches QM-Harz enthalten, welches erstens einen Vinylgruppengehalt von 0,5 - 8 mMol/g besitzt und zweitens aus SiO_{4/2}-, RO_{1/2}- und R₃SiO_{1/2}-Einheiten besteht und R für eine Methyl-, Vinyl-, Phenyl- und Ethyl-Gruppe steht und einen Ethoxygruppengehalt von weniger als 4 mMol/g aufweist.

Komponente b) der oben genannten Rezeptur ist in der Basispaste, Komponente c) in der Katalysatorpaste enthalten. Basis- und Katalysatorpaste des erfindungsgemäßen Bißregistriermaterials zeichen sich durch hohe Transparenz und gute Lagerstabilität hinsichtlich Viskosität aus; die daraus hergestellten Bißregistrate weisen Härte, hohe Transparenz und Reißfestigkeit bei geringer elastischer Verformung auf.
Bei dem Organopolysiloxan a) handelt es sich um an sich bekannte vinylendgestoppte Polydimethylsiloxane, deren Viskosität im Bereich von 100 bis 100.000 mPa·s, vorzugsweise 200 bis 10.000 mPa·s bei 20°C liegt(Silikonöle).

Die als Vernetzer fungierenden organohydrogenpolysiloxane sind b) sind ebenfalls an sich bekannte Polydimethylsiloxane, welche im Molekül Wasserstoffatome an mindestens zwei Siliziumatomen aufweisen.

Bei dem Katalysator c) handelt es sich um einen Platinkomplex, der aus Hexachlorplatinsäure hergestellt wurde. Auch diese Verbindungen sind an sich bekannt.

Die Farbstoffe d), die gegebenfalls zur Unterscheidung von Basis- und Katalysatorpaste und zur Mischkontrolle eingesetzt werden, sollten feindispers sein, um die Transparenz nicht zu stören. Neben anorganischen werden organische Farbpigmente bevorzugt. Besonders bevorzugt können gemäß DE-A-35 44 619 Lösungen von Farbstoffen in Isoparaffinen wie 2,2,4,4,6,6,8-Heptamethylnonan (C₁₆H₃₄) oder 2,2,4,4,6,6,8,8,10-Nonamethylundecan (C₂₀H₄₂) verwendet werden.

Die erfindungsgemäß einzusetzenden Compounds e) werden z.B., wie in der DE-A-25 35 324 beschrieben, aus einer gefällten oder pyrogen hergestellten Kieselsäure mit einer spezifischen Oberfläche von mehr als 50 m²/g nach BET und einem trimethylsilyl- und/oder vinylendgestoppten Polydimethylsiloxan mit einer Viskosität bevorzugt zwischen 100 und 200.000 mPa·s bei 20°C unter Verwendung von Modifizierhilfmitteln, z.B. Hexarmethyldisilazan, Tetramethyldivinyldisilazan hergestellt.

Man geht dabei so vor, daß man den Füllstoff während des Einarbeitungsprozesses in Gegenwart von Wasser mit Modifizierungsmitteln der allgemeinen Formel

(R₃Si)ₙ-X

wobei R für einen substituierten oder nicht substituierten gesättigten oder aliphatisch ungesättigten Kohlenwasserstoffrest mit bis zu 10 C-Atomen steht, X Halogen, H, OH, OR, S, OOCR, N oder NY (Y = Wasserstoff oder R) ist und n 1, 2 oder 3 bedeutet, behandelt.

Die für die erfindungsgemäßen Massen geeigneten Compounds enthalten dir folgenden Einzelbestandteile:
1. 20 bis 1000, vorzugsweise 50 - 200 Gewichtsteile eines linearen Polyorganosiloxans der allgemeinen Formel 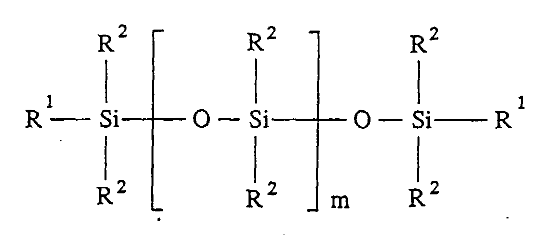 wobei R² gleich oder verschiedene einwertige substituierte oder nichtsubstituierte Kohlenwasserstoffreste darstellt, R¹ dieselbe Bedeutung wie R² hat und darüber hinausgehend einen Vinylrest darstellen kann und m eine ganze positive Zahl ist;
2. 5 bis 500, vorzugsweise 10 - 100 Gewichtsteile eines hochdispersen aktiven Füllstoffes mit einer BET-Oberfläche von mindestens 50 m²/g.

Bei dem oben angegebenen Bestandteil 1) handelt es sich um ein lineares Polyorganosiloxan, dessen Molekülkette mit einwertigen Kohlenwasserstoffresten R² abgesättigt ist. R² kann ein substituierter oder nichtsubstituierter Kohlenwasserstoffrest sein und ist vorzugsweise ausgewählt aus Alkylresten, wie z.B. Methyl, Ethyl, Propyl, Butyl, Hexyl, i-Propyl, Amyl, oder Arylresten, wie z.B. Tolyl, Xylyl, Ethylphenyl, oder Aralkylreste, wie z.B. Benzyl, Phenylethyl oder halogensubstituiertesn Alkyl- oder Arylresten, wie z.B. Chlormethyl, 3,3,3-Trifluorpropyl, Chlorphenyl, Tetrachlorphenyl, Difluorphenyl und Alkenylresten, wie z.B. Vinyl, Allyl. Außerdem steht R² auch für Cyanalkyl-, Cycloalkyl- und Cycloalkenylreste.

R¹ hat dieselbe Bedeutung wie R², kann darüber hinaus aber auch eine Vinylgruppe darstellen.

In einem Molekül des Bestandteiles 1) können unterschiedliche Reste R¹ und R² vorhanden sein.

Die Größe der Zahl m ist maßgebend für die Viskosität des linearen Polyorganosiloxans und ist so gewählt, daß die Viskosität des linearen Polyorganosiloxans bevorzugt zwischen 100 mPa·s und 2.10⁵ mPa·s bei 20 C liegt. Bestandteil 1) kann auch aus Mischungen von Polymeren mit unterschiedlicher Größe der Zahl m bestehen.

Komponente 2) ist ein handelsüblicher hochdisperser, aktiver Füllstoff mit einer BET-Oberfläche von mindestens 50 m²/g. In Frage kommen Stoffe wie TiO₂, Al₂O₃, ZnO oder bevorzugt pyrogen gewonnene oder naßgefällte Kieselsäure. Das Herstellungsverfahren und die chemische Natur des Füllstoffes spielen für die erfindungsgemäße Verwendung keine Rolle. Wichtig ist lediglich, daß es sich um einen Füllstoff mit einer BET-Oberfläche in der angegebenen Größenordnung handelt. Vorteilhaft ist ferner, daß kein während oder nach seiner Herstellung modifizierter Füllstoff verwendet wird. Beispielhaft als geeignete Füllstoffe seien genannt pyrogen gewonnene oder naßgefällte Kieselsäure, die unter den Handelsnamen AEROSIL® (Degussa), CABOSIL® (Cabot), HDK® (Wacker-Chemie), pyrogen gewonnenes Aliminiumoxid oder Titandioxid unter der Bezeichnung ALUMINIUMOXID O® bzw. TITANDIOXID P 25® (Degussa) oder ZINKOXID AKTIV® (BAYER AG) angeboten werden.

Die Einarbeitung des hochdispersen aktiven Füllstoffs geschieht gemäß DE-A-25 35 334 in der Weise, daß der Füllstoff während des Einarbeitungsprozesses in Gegenwart von Bestandteil 1) in geeigneter Form an der Oberfläche modifiziert wird. Dazu ist es notwendig, daß vor der Zugabe des Füllstoffs des Modifizierungsmittel zu Bestandteil 1) zugefügt wird. Bei dem Modifizierungsmittel handelt es sich um eine Substanz, die in der Lage ist, die Oberfläche des Füllstoffs ohne Gegenwart eines zusätzlichen Katalysators in der gewünschten Weise zu modifizieren, die aber nicht mit Bestandteil 1) unter den gewählten Reaktionsbedingungen eine chemische Reaktion eingeht. Es wurde festgestellt, daß Verbindungen der allgemeinen Formel

(R₃Si)ₙX

geeignet sind, Füllstoffe in der gewünschten Weise in Gegenwart von Bestandteil 1) zu modifizieren. Dabei hat R die oben angegebenen Bedeutung, n ist eine positive Zahl und kann den Wert 1, 2 oder 3 haben und X ist ein Rest der Formel H, OH, OR, Halogen, S, OOCR, N oder NY (wobei R unter a) angegebene Bedeutung hat und Y ein einwertiger Kohlenwasserstoffrest oder Wasserstoff ist). Beispielhaft seien genannt: Hexamethyldisilazan, Trimethylsilan, Trimethylchlorsilan, Trimethylethoxysilan, Triorganosilylacylate oder Triorganosilylamine.

Besonders bevorzugt sind erfindungsgemäß Verbindungen, in denen der Rest R ein Methylrest ist und der Rest X die Gruppe NY darstellt, wobei Y vorzugsweise ein Wasserstoffatom ist.

In der Regel wird das Modifizierungsmittel in einer Menge von 1-50, vorzugsweise 3-20 Gewichtsteilen zugegeben.

Es ist bevorzugt, daß zu der Mischung von Bestandteil 1) und dem Modifizierungsmittel vor Zugabe des hochdispersen aktiven Füllstoffes Wasser in einer Menge von 0,1 bis 10 Gewichtsteilen, vorzugsweise von 2 bis 6 Gewichtsteilen zugefügt wird. Durch die Gegenwart von Wasser wird die Reaktion zwischen Modifizierungsmittel und hochdispersen Füllstoff gegünstigt. Zusätzlich trägt das Wasser zu einer beschleunigten gleichmäßigen Verteilung des Füllstoffes in Gegenwart des Modifizierungsmittels bei.

Die Einarbeitungszeit des hochdispersen aktiven Füllstoffes erfolgt bei Raumtemperatur oder nur leicht erhöhter Temperatur. Die Einarbeitung ist unkritisch, und es brauchen keine besonderen Vorsichtmaßnahmen berücksichtigt zu werden. Es ist zweckmäßig, den hochdispersen aktiven Füllstoff zu der Mischung aus Bestandteil 1), dem Modifizierungsmittel und Wasser nicht auf einmal, sondern portionsweise zuzugeben, so daß die jeweils zugefügte Menge an hochdispersen aktivem Füllstoff in kurzer Zeit benetzt und eingearbeitet wird. Die Verteilung des hochdispersen aktiven Füllstoffes in der Mischung aus Bestandteil 1), dem Modifizierungsmittel und Wasser kann mit handelsüblichen, dafür geeigneten Geräten erfolgen, vorzugsweise mit sogenannten Z-Knetem oder Planetenrührern.

Die Menge des einzuarbeitenden hochdispersen aktiven Füllstoffs richtet sich nach der gewünschten Konsistenz der Mischung und im Falle von Mischungen, in denen Bestandteil 1) Reste R¹ oder R² enthält, die einer Vernetzungsreaktion bei Raumtemperatur mit entsprechenden Vernetzersubstanzen zugänglich sind, nach den erforderlichen mechanischen Eigenschaften der vernetzten Gummis.

Nach vollständiger Einarbeitung des hochdispersen aktiven Füllstoffes wird das aus Komponente 1), dem Modifizierungsmittel, Wasser und Füllstoff bestehende Gemisch vorzugsweise einer kurz währenden mechanischen Beanspruchung (z.B. Überdruck, Druck zwischen Walzen, Knetbeanspruchung) in der Weise unterworfen, daß das Gemisch 10 Minuten bis 2 Stunden lang im dicht verschlossenen Mischgerät verbleibt. Nach Beendigung der mechanischen Beanspruchung der Mischung wird überschüssiges Modifizierungsmittel und Wasser in der Weise entfernt, daß entweder Vakuum angelegt wird oder das Mischaggregat bei erhöhter Temperatur so lange geöffnet und abgelüftet wird, bis überschüssiges Modifizierungsmittel und Wasser sich praktisch vollständig verflüchtigt haben. Vorzugsweise werden gleichzeitig die Temperatur erhöht und Vakuum angelegt.

Die erfindungsgemäß einzusetzenden kurzkettigen vinylendgestoppten Polydimethylsiloxane f) sind an sich bekannt. So werden in DE-A-26 46 726 Verbindungen beschrieben mit der allgemeinen Formel

CH₂=CH-R₂ SiO-(SiR₂O)ₙ-SiR₂-CH=CH₂

worin R gleiche oder verschiedene, von aliphatischen Mehrfachbindungen freie, einwertige, gegebenfalls substituierte Kohlenwasserstoffreste und n 0 oder eine ganze Zahl im Wert von 1 bis 6 bedeutet. Diese Verbindungen werden in Mengen von 1 bis 5000 Gewichts-ppm, bezogen auf das Gesamtgewicht aller Einsatzstoffe, als Inhibitoren zum Regeln der Vernetzungsgeschwindigkeit von additionsvernetzenden Silikonabformmassen eingesetzt

Überraschenderweise wurde nun gefunden, daß Verbindungen gleicher o. g. allgemeiner Formel, worin R die gleiche o. g. Bedeutung hat und n aber eine ganze Zahl von 10 bis 20 bedeutet, keineswegs inhibierend wirken und in einer Menge von 1 bis 8 Gewichtsprozent, bezogen auf die Gesamtmenge, eingesetzt werden können bei kurzen Reaktionszeiten der Mischung. Die vernetzenden Gummis weisen eine höhere Härte auf.

Die gegebenenfalls erfindungsgemäß eingesetzten QM-Harze g) sind an sich bekannt. Ihre Herstellung ist z. B. in den US PS 2676 182, 2 857 356 und 3 527 659 beschrieben.

Wesentlich für den erfindungsgemäßen Einsatz ist,
- daß die QM-Harze so niedermolekular sind, daß sie in den vinylendgestoppten Polydimethylsiloxanen optisch klar gelöst sind und ihre Abmischungen zu optisch klaren Endprodukten führen. In der Regel weisen derartige QM-Harze eine Viskosität von 150 bis 1500 mPa·s bei 20°C auf;
- daß der SiOH-Gehalt der QM-Harze so gering ist, daß keine optischen Störungen durch Blasen von freigesetztem Wasserstoffgas entstehen können;
- daß der Gehalt an flüchtigen und/oder nicht reaktiven Molekülen so gering ist, daß die Dimensionsstabilität der Formkörper nicht gemindert wird; und daß die QM-Harze einen Vinylgehalt von 0,5 bis 8 mMol/g und einen Ethoxy-Gehalt von 0,2 bis 3 mMol/g aufweisen.

Das Verhältnis der Summe aus R₃SiO_{1/2}- und EtO_{1/2}-Einheiten zu den SiO₄/2-Einheiten soll dabei kleiner als 2,5 sein und wird nach unten begrenzt durch die optisch klare Löslichkeit der QM-Harze in den vinylendgestoppten Polydimethylsiloxanen, bzw. durch die optische Klarheit der Endprodukte.

Die QM-Harze können darüber hinaus geringe Anteile an R₂SiO- und RSiO_{3/2}-Einheiten enthalten, ohne daß dies den erfindungsgemäßen Einsatz stört, jedoch sollen die R₂SiO- und RSiO_{3/2}-Einheiten in Summe 20 MolGew.-% nicht überschreiten.

Ein bevorzugtes QM-Harz weist die Formel

[SiO_{4/2}][(CH₃)₂CH₂=CHSiO_{1/2}]_{0,9-1,4}[C₂H₅O_{1/2}]_{0,4-0,5}

auf; besitzt einen Vinylgehalt von 5,5 - 6,5 mMol/g und eine Viskosität von 500 - 800 mPa·s bei 23°C.

Vorzugsweise enthalten erfindungsgemäße Bißregistrierungsmaterialien (d. h. das Gemisch aus Basis- und Katalysatorpaste) folgende Mengenanteile (in Gew.-% bezogen auf die gesamte Paste) der einzelnen Komponenten:
a) 5 - 30 Gew.-%, insbesondere 10 - 25 Gew.-% des vinylgruppenhaltigen Polysiloxans
b) 1 - 10 Gew.-%, insbesondere 2 - 6 Gew.-% Vernetzer
c) 0,005 - 2 Gew.-%, insbesondere 0,01 bis 1 Gew.-% Katalysator
e) 0 - 1 Gew.-%, insbesondere 0 bis 0,5 Gew.-% Farbstoffe
d) 30 - 80 Gew.-%, insbesondere 50 - 75 Gew.-% des erfindungsgemäß zu verwendeten Compounds
f) 1 - 10 Gew.-%, insbesondere 2 - 6 Gew.-% des erfindungsmäßigen kurzkettigen vinylgruppenhaltigen Polysiloxans und
g) 0 - 10 Gew.-% insbesondere 0 - 5 Gew.-% des erfindungsmäßigen kurzkettigen vinylgruppenhaltigen QM-Harzes

Vorzugsweise enthalten die erfindungsgemäß zu verwendenden Compounds 5 - 50 Gew.-% (bezogen auf gesamtes Compound), insbesondere 15 - 40 Gew.-% an hochdispersen aktiven Füllstoffen.

Die nachfolgenden Beispiele, in denen alle Teile Gewichtsteile bedeuten, erläutern die Erfindung.

### Beispiel 1

In einem Kneter werden 270 Teile vinylendgestopptes Polydimethylsiloxan mit einer Viskosität von 10 Pa.s und 310 Teile vinylendgestopptes Polydimethylsiloxan mit einer Viskosität von 65 Pa^{.}s bei 20°C mit 68 Teilen Hexamethyldisilazan und 12 Teilen Tetramethyldivinyldisilazan und 50 Teile Wasser gemischt und anschließend mit 330 Teilen einer pyrogenen Kieselsäure einer BET-Oberfläche von 300 m²/g zu einer homogenen Masse verknetet. Die Mischung wurde zunächst auf 130°C erwärmt und 1,5 Stunden im geschlossenen Kneter gerührt und danach bei 160° C unter Vakuum 1 Stunde von leichtflüchtigen Bestandteilen befreit. Nach dem Erkalten wird der Compound mit 120 Teilen vinylendgestopptem Polydimethylsiloxan mit einer Viskosität von 10 Pa·s bei 20 C unter Rühren verdünnt.

### Beispiel 2

Die Basispaste eines Bißregistriermaterials wurde hergestellt in einem Kneter durch Vermischen von 70,0 Teilen Compound aus Beispiel 1, 6,6 Teilen eines SiH-gruppenhaltigen Polydimethylsiloxans mit einen SiH-Gehalt von 7,5 mMol/g und einer Viskosität von 60 mPa·s bei 20°C sowie 23,4 Teilen eines vinylendgestoppten Polydimethylsiloxans mit einer Viskosität von 200 mPa·s bei 20°C.

Die dazugehörende Katalysatorpaste wurde erhalten durch Vermischen von 69,2 Teilen Compounds aus Beispiel 1, 0,8 Teilen eines Komplexes aus Platin und Divinyltetramethyldisiloxan als Katalysator, 10 Teilen des kurzkettigen vinylendgestoppten Polydimethylsiloxans mit einen SiVi-Gehalt von 1,8 mMol/g und einer Viskosität von 10 mPa·s bei 20°C sowie 20 Teilen eines vinylendgestoppten Polydimethylsiloxans mit einer Viskosität von 200 mPa·s bei 20°C.

Beide Pasten hatten eine Viskosität von ca. 50.000 mPa·s bei 20°C und waren transparent. Sie wurden jeweils in eine Kammer einer Doppelkartusche gefüllt und mittels einer Doppelkolbenpistole durch einen aufgesetzten Statikmischer gedrückt und damit gemischt. 30 Sekunden nach Verlassen des Statikmischers war die Verarbeitungszeit durch Anstieg der Viskosität infolge der einsetzenden Additionsreaktion beendet, nach weiteren 2 Minuten war die Mischung zu einem transparenten Gummi mit guter Reißfestigkeit und einer Härte von 69 Shore A vernetzt. Dieses Bißregistriermaterial wurde in der anwendungstechnischen Prüfung gut beurteilt.

### Beispiel 3

Die Herstellung einer Bißregistriermaterial-Basispaste erfolgt in einem Kneter durch Vermischen von 67,5 Teilen Compound aus Beispiel 1, 24,0 Teilen eines SiH-gruppenhaltigen Polydimethylsiloxans mit einem SiH-Gehalt von 7,5 mMol/g und einer Viskosität von 60 mPa·s bei 20°C sowie 8,5 Teilen eines vinylendgestoppten Polydimethylsiloxans mit einer Viskosität von 200 mPa·s bei 20°C.

Die dazugehörende Katalysatorpaste wurde in gleicher Weise hergestellt durch Vermischen von 70,0 Teilen Compound aus Beispiel 1, 15,2 Teilen eines vinylendgestoppten Polydimethylsiloxans mit einer Viskosität von 200 mPa·s bei 20°C, 9,0 Teilen eines kurzkettigen, vinylendgestoppten Polydimethylsiloxans mit einem SiVi-Gehalt von 1,8 mMol/g und einer Viskosität von 10 mPa·s bei 20°C, 5,0 Teilen eines QM-Harzes der Formel

[SiO_{4/2}][Me Vi SiO_{1/2}]_{1,0} [Et O_{1/2}]_{0,4}

mit einer Viskosität von 800 mPa·s bei 20°C sowie 0,8 Teilen eines Komplexes aus Platin und Divinyltetramethyldisiloxan als Katalysator.

Die Viskosität der Basispaste lag bei ca. 47.000 mPa·s und der Katalysatorpaste bei ca. 52.000 mPa·s bei 20°C. Beide Pasten waren transparent. Die wie oben beschrieben vermischten Pasten hatten eine Verarbeitungszeit von 45 Sekunden und eine Abbindezeit von 2 Minuten 45 Sekunden. Der vernetzte Gummi war transparent, wies eine hohe Reißfestigkeit und eine Härte von 78 Shore A auf. Die anwendungstechnische Beurteilung dieses Bißregistriermaterials war sehr gut.

### Beispiel 4 (Vergleich)

In einem Kneter wurde die Basispaste eines Bißregistriermaterials hergestellt durch Vermischen von 70,0 Teilen Compound aus Beispiel 1, 5,8 Teilen eines SiH-gruppenhaltigen Polydimethylsiloxans mit einem SiH-Gehalt von 7,5 mMol/g und einer Viskosität von 60 mPa·s bei 20°C sowie 24,2 Teilen eines vinylendgestoppten Polydimethylsiloxans mit einer Viskosität von 200 mPa·s bei 20°C.

Die Herstellung der entsprechenden Katalysatorpaste erfolgte in einem Kneter durch Vermischen von 76,4 Teilen Compound aus Beispiel 1, 0,6 Teilen eines Komplexes aus Platin und Divinyltetramethyldisiloxans als Katalysator und 24,0 Teilen eines vinylendgestoppten Polydimethylsiloxans mit einer Viskosität von 200 mPa^{.}s bei 20°C.

Die Basispaste hatte eine Viskosität von ca. 48.000 mPa·s, die Katalysatorpaste ca. 58.000 mPa·s bei 20°C. Beide Pasten waren transparent. Die Pasten in eine Doppelkartusche gefüllt und mittels Doppelkolbenpistole durch den aufgesetzten Statikmischer gedrückt ergaben eine Verarbeitungszeit von 40 Sekunden und eine Abbindezeit von 2 Minuten 30 Sekunden. Der vernetzte Gummi hatte eine gute Transparenz und Reißfestigkeit, wies aber nur eine Shore A-Härte von 50 auf und war sehr flexibel. Die anwendungstechnische Prüfung beurteilte das Material wegen seiner geringen Härte und hohen Flexibilität als wenig geeignet.

### Beispiel 5 (Vergleich)

Die Basispaste eines Bißregistrierungsmaterials wurde in einem Kneter hergestellt durch Vermischen von 10,0 Teilen Compound aus Beispiel 1, 5,8 Teilen eines SiH-gruppenhaltigen Polydimethylsiloxans mit einem SiH-Gehalt von 7,5 mMol/g und einer Viskosität von 60 mPa·s bei 20°C, 24,0 Teilen eines vinylendgestoppten Polydimethylsiloxans mit einer Viskosität von 1.000 mPa·s bei 20°C, 59,8 Teilen Cristobalitfeinstmehl mit einer mittleren Korngröße von ca. 4 µm und 0,4 Teilen eines organischen Farbpigments.

Die dazugehörende Katalysatorpaste wurde erhalten durch Vermischen von 16,5 Teilen Compound aus Beispiel 1, 23,0 Teilen eines vinylendgestoppten Polydimethylsiloxans mit einer Viskosität von 1.000 mPa·s bei 20°C, 0,5 Teilen eines Komplexes aus Platin und Divinyltetramethyldisiloxan und 60,0 Teilen o. g. Cristobalitfeinstmehl.

Die Basispaste hatte eine Viskosität von ca. 54.000 mPa·s und die Katalysatorpaste von ca. 60.000 mPa·s bei 20°C, beide Pasten waren opak. Die wie oben beschrieben vermischten Pasten hatten eine Verarbeitungszeit von 45 Sekunden und eine Abbindezeit von 2 Minuten 45 Sekunden. Der vernetzte Gummi hatte eine geringe Reißfestigkeit, eine Härte von 77 Shore A und war nicht durchsichtig. Dieses Bißregistriermaterial wurde in der anwendungstechnischen Prüfung wegen seiner Lichtundurchlässigkeit und leichten Sprödigkeit als mäßig geeignet beurteilt.

### Vergleichsbeispiel 6 (gemäß US 4,571,188)

94 Gew.-% Vinyl endgestopptes Polydimethylsiloxan mit einer Viskosität von 65.000 mPa·s und 6 % eines Vernetzers aus 70 Gew.-% Dimethyl- und 30 Gew.-% Methylhydrogensiloxan werden zu einer Basispaste vermischt. Eine Katalysatorpaste wird aus 99,8 Gew.-% Vinyldimethyl endgestopptem Polydimethylsiloxan einer Viskosität von 65.000 mPa·s und 0,2 Gew.-% Platinkatalysator hergestellt.

Die Mischung hat eine Verarbeitungszeit von 20 Sekunden und eine Vemetzungszeit von 1 Minute. Das erhaltene vernetze Produkt war jedoch in Folge zahlreicher eingeschlossener Gasblasen nicht ausreichend transparent.

### Beispiel 7 (Vergleich)

Um die gemäß Beispiel 6 erhaltenen negativen Eigenschaften hinsichtlich Transparenz des Materials wurden die Komponenten mit einer Doppelkolbenpistole in einem Statikmixer gemischt. Die Verarbeitungszeit war 22 Sekunden, die Vemetzungszeit 1 Minute. Die Härte (Shore A) war jedoch nur 18, das Produkt war zwar transparent, hatte aber keine ausreichende Zugfestigkeit.

### Beispiel 8 (Vergleich)

Beispiel 7 wurde modifiziert, indem die Basispaste aus 96 Gew.-% des Vinyl endgestoppten Polydimethylsiloxans und 4 Gew.-% des Crosslinkers gemäß Beispiel 6 hergestellt wurde. Die Zusammensetzung des Katalysators war die gleiche.

Die Verarbeitungszeit war 30 Sekunden, die Vemetzungszeit 1 Minute und 2C Sekunden, die Härte (Shore A) war 19. Das erhaltene Produkt verfügte zwar über eine ausreichende Transparenz, jedoch war die Zugfestigkeit völlig unzureichend.

### Beispiel 9 (Vergleich)

Die oben beschriebene Mischung wurde erneut modifiziert, indem die Basispaste aus 94 Gew.-% des gleichen Vinyldimethyl endgestoppten Polydimethylsiloxans und 6 Gew.-% des Vernetzers aus 90 Gew.-% Dimethyl- und 10 Gew.-% Methylhydrogensiloxan hergestellt wurde. Die Katalysatorpaste wurde aus 97 Gew.-% Vinyldimethyl endgestopptem Polydimethylsiloxan, wie oben beschrieben und 3 Gew.-% Platinkatalysator hergestellt.

Die Verarbeitungszeit war 2 Minuten und 30 Sekunden, die Vemetzungszeit ca. 6 Minuten. Die Härte (Shore A) war 18, die Transparenz des Produktes war zwa ausreichend, jedoch lag die Zugfestikeit des Materials erneut unter den Mindestanforderungen der Anwendung.

Abgesehen davon war die Viskosität der Mischung vor der Vernetzung zu niedrig für dentale Anwendungen. Die Mischung würde bei dem Versuch sie anzuwenden, von den Zähnen in den Kiefer- und Rachenbereich des Patienten fließen können.

### Beispiel 10

Entsprechend der vorliegenden Erfindung wurde Beispiel 3 durch den Einsatz einer Katalysatorkomponente aus 2,5 Gew.-% des QM-Harzes anstelle von 5 Gew.-% desselben modifiziert. Die Verarbeitungszeit war 45 Sekunden, die Vernetzungszeit 3 Minuten. Die Härte (Shore A) war 73, das Produkt war ausreichend transparent und von einer hohen Zugfestigkeit.

Die vorstehenden Beispiele zeigen deutlich die Überlegenheit der erfindungsgemäßen Zusammensetzungen gegenüber denen des Standes der Technik. Letztere weisen eine völlig unzureichende Zugfestigkeit auf, wodurch sie nicht ohne Beschädigung von den zu behandelnde Zähnen entfernt werden können. Daher können derartige Zusammensetzungen nicht verwendet werden, um vor der Versorgung einer Kavität mit einem lichthärtenden Komposit einen sogenannten Okklusalstempel des zu versorgenden Bereiches herzustellen.

## Patentansprüche

1. Transparente additionsvernetzende Massen auf Polysiloxanbasis, enthaltend
a) 5 - 30 Gew.-% Organopolysiloxane mit zwei oder mehreren Vinylgruppen im Molekül und einer Viskosität zwischen 100 und 100.000 mPa -s bei 20°C,
b) 1 - 10 Gew.-% Organohydrogenpolysiloxane als Vernetzer,
c) 0.005 - 2.0 Gew.-% eines Katalysators zur Beschleunigung der Additionsreaktion und gegebenenfalls
d) bis zu 1 Gew.-% Farbstoffen,
dadurch gekennzeichnet, daß die Massen zusätzlich
e) 30 - 80 Gew.-% eines Compounds aus 95 - 50 Gew.-% eines vinylendgestoppten Polydimethylsiloxans mit einer Viskosität zwischen 100 und 200.000 mPa -s bei 20°C und 5 - 50 Gew.-% einer gefällten oder pyrogen hergestellten Kieselsäure mit einer spez. Oberfläche von mindestens 50 m²/g nach BET, und
f) 1 - 10 Gew.-% Organopolysiloxane der allgemeinen Formel
CH₂=CH-R₂SiO-(SiR₂O)ₙSiR₂- CH=CH₂,
worin
R gleiche oder verschiedene, von aliphatischen Mehrfachbindungen freie, einwertige, gegebenenfalls substituierte Kohlenwasserstoffreste, und
n eine ganze Zahl im Wert von 10 bis 20 bedeutet, und
g) 0 - 10 Gew.-% eines niedermolekularen, vinyl- und ethoxygruppenhaltigen, in a) homogen löslichen QM-Harzes, welches einen Vinylgruppengehalt von 0,5 - 8 mMol/g besitzt und aus SiO_{4/2}-, RO_{1/2}- und R₃SiO_{1/2}-Einheiten besteht, worin R für eine Methyl-, Vinyl-, Phenyloder Ethyl-Gruppe steht, und einen Ethoxygruppengehalt von weniger als 4 mMol/g aufweist.

2. Massen nach Anspruch 1, dadurch gekennzeichnet, daß der Compound e) in Gegenwart von Wasser und Hexamethylsilazan als Modifizierungsmittel hergestellt wurde.

3. Massen nach Ansprüchen 1 und 2, dadurch gekennzeichnet, daß sie
a) 10 bis 25 Gew.-% des vinylgruppenhaltigen Polysiloxans,
b) 2 bis 6 Gew.-% Vemetzer,
c) 0,01 bis 1,0 Gew.-% Katalysator,
d) 0 bis 0,5 Gew.-% Farbstoffe,
e) 50 bis 75 Gew.-% Compound,
f) 2 bis 6 Gew.-% des kurzkettigen vinylgruppenhaltigen Polysiloxans und
g) 0 bis 5 Gew.-% des kurzkettigen vinylgruppenhaltigen QM-Harzes
enthalten.

4. Verwendung der Massen nach Ansprüchen 1 bis 3 als Bißregistriermaterial, Okklusalstempel oder Fixierungsschiene.

5. Verwendung der Massen nach Ansprüchen 1 bis 3 als Bißregistriermaterial.

6. Verwendung der Massen nach Ansprüchen 1 bis 3 als Okklusalstempel zur Formung von Seitenzahncompositen und als Fixierungsschiene für Brackets in der Kieferorthopädie.

## Claims

1. Transparent, polysiloxane-based compositions which cross-link by an addition reaction, containing
a) from 5 to 30 wt.% organopolysiloxanes having two or more vinyl groups in the molecule and a viscosity of between 100 and 100,000 mPa.s at 20°C,
b) from 1 to 10 wt.% organohydrogen polysiloxanes as cross-linking agents,
c) from 0.005 to 2.0 wt.% of a catalyst to accelerate the addition reaction and optionally
d) up to 1 wt.% dyes,
characterised in that the compositions contain additionally
e) from 30 to 80 wt.% of a compound comprising from 95 to 50 wt.% of a vinyl-terminated polydimethyl siloxane having a viscosity of between 100 and 200.000 mPa.s at 20°C and from 5 to 50 wt.% of a silica prepared by precipitation or pyrogenically and having a BET specific surface area of at least 50 m²/g, and
f) from 1 to 10 wt.% organopolysiloxanes of the general formula
CH₂=CH-R₂SiO-(SiR₂O)ₙSiR₂-CH=CH₂,
in which
R denotes monovalent hydrocarbon radicals which are free of aliphatic multiple bonds, are optionally substituted and may be the same or different, and
n denotes an integer from 10 to 20, and
g) from 0 to 10 wt.% of a low molecular weight QM resin which contains vinyl groups and ethoxy groups and is soluble in homogeneous manner in a), and which has a vinyl group content of from 0.5 to 8 mmol/g, comprises SiO_{4/2}, RO_{1/2} and R₃SiO_{1/2} units, in which R stands for a methyl, vinyl, phenyl or ethyl group, and exhibits an ethoxy group content of less than 4 mmol/g.

2. Compositions according to Claim 1, characterised in that the compound e) has been prepared in the presence of water using hexamethyl silazane as a modifier.

3. Compositions according to Claims 1 and 2, characterised in that they contain
a) from 10 to 25 wt.% of the vinyl group-containing polysiloxane,
b) from 2 to 6 wt.% cross-linking agents,
c) from 0.01 to 1.0 wt.% catalyst,
d) from 0 to 0.5 wt.% dyes,
e) from 50 to 75 wt.% compound,
f) from 2 to 6 wt.% of the short-chain vinyl group-containing polysiloxane and
g) from 0 to 5 wt.% of the short-chain vinyl group-containing QM resin.

4. Use of the compositions according to Claims 1 to 3 as an occlusion recording material, occlusal stamp or fixing strip.

5. Use of the compositions according to Claims 1 to 3 as an occlusion recording material.

6. Use of the compositions according to Claims 1 to 3 as an occlusal stamp for forming composites for posterior teeth and in orthodontics as a bracket fixing strip.

## Revendications

1. Matières transparentes réticulant par addition, à base de polysiloxane, contenant
a) 5 - 30 % en poids d'organopolysiloxanes portant deux ou plusieurs groupes vinyle par molécule et d'une viscosité comprise entre 100 et 100.000 mPa.s à 20 °C,
b) 1 - 10 % en poids d'organohydrogénopolysiloxanes utilisés comme réticulants,
c) 0,005 - 2,0 % en poids d'un catalyseur destiné à accélérer la réaction d'addition, et facultativement
d) jusqu'à 1 % en poids de colorants,
caractérisées en ce que ces matières contiennent en plus
e) 30 - 80 % en poids d'un matériau composite à base de 95 - 50 % en poids d'un polydiméthylsiloxane à terminaison vinyle, de viscosité comprise entre 100 et 200.000 mPa.s à 20 °C et 5 - 50 % en poids d'un acide silicique précipité ou pyrogéné de surface spécifique inférieure à 50 m²/g selon la méthode de Brunauer-Emmett-Teller, et
f) 1 - 10 % en poids d'organopolysiloxanes de formule générale
CH₂=CH-R₂SiO-(SiR₂O)ₙSiR₂-CH=CH₂,
où
R désigne des restes hydrocarbure identiques ou différents, monovalents, sans liaisons multiples aliphatiques, facultativement substitués, et
n désigne un nombre entier d'une valeur de 10 à 20, et
g) 0 - 10 % en poids d'une résine QM de faible poids moléculaire, formant une solution homogène dans a) et contenant des groupes vinyle et éthoxy, dont la teneur en groupes vinyle est de 0,5 - 8 mmoles /g et qui est composée d'unités SiO_{4/2}, RO_{1/2} et R₃SiO_{1/2}, R étant ici un groupe méthyle, vinyle, phényle ou éthyle, et qui présente une teneur en groupes éthoxy inférieure à 4 mmoles/g.

2. Matières selon la revendication 1, caractérisées en ce que le matériau composite e) a été préparé en présence d'eau et d'hexaméthylsilazane utilisé comme agent modifiant.

3. Matières selon les revendications 1 et 2, caractérisées en ce qu'elles contiennent
a) 10 à 25 % en poids du polysiloxane contenant des groupes vinyle,
b) 2 à 6 % en poids de réticulant,
c) 0,01 à 1,0 % en poids de catalyseur,
d) 0 à 0,5 % en poids de colorants,
e) 50 à 75 % en poids de matériau composite,
f) 2 à 6 % en poids du polysiloxane à chaîne courte contenant des groupes vinyle, et
g) 0 à 5 % en poids de la résine QM à chaîne courte contenant des groupes vinyle.

4. Utilisation des matières selon les revendications 1 à 3 comme matériau de prise d'empreintes de l'articulé dentaire, empreinte occlusale ou attelle de fixation.

5. Utilisation des matières selon les revendications 1 à 3 comme matériau de prise d'empreintes de l'articulé dentaire.

6. Utilisation des matières selon les revendications 1 à 3 comme empreinte occlusale pour le moulage de reconstitutions dentaires latérales composées et comme attelle de fixation pour crochets en orthopédie de la mâchoire.
